Europäisches Patentamt

European Patent Office

Office européen des brevets

(19)

(11) Publication number : **0 516 443 A1**

## (12) EUROPEAN PATENT APPLICATION

(21) Application number : **92304902.7**

(22) Date of filing : **29.05.92**

(51) Int. Cl.$^5$ : **C12Q 1/66, C12N 15/65, C12N 1/21, C12N 9/02, C12N 15/70, C07K 15/16**

The applicant has filed a statement in accordance with Rule 28 (4) EPC (issue of a sample only to an expert). Accession number(s) of the deposit(s): NRRL B-18783 and NRRL B-18784.

(30) Priority : **31.05.91 US 708548**

(43) Date of publication of application :
**02.12.92 Bulletin 92/49**

(84) Designated Contracting States :
**AT BE CH DE DK ES FR GB GR IT LI LU NL PT SE**

(71) Applicant : **ELI LILLY AND COMPANY**
**Lilly Corporate Center**
**Indianapolis Indiana 46285 (US)**

(72) Inventor : **Gadski, Robert Alan**
**4431 North Illinois Street**
**Indianapolis, Indiana 46208 (US)**
Inventor : **McClure, Don B.**
**7816 Mallard Way**
**Indianapolis, Indiana 46256 (US)**
Inventor : **Schmidt, Robert John**
**8453 Slippery Elm Court**
**Indianapolis, Indiana 46227 (US)**

(74) Representative : **Hudson, Christopher Mark et al**
**Erl Wood Manor**
**Windlesham Surrey GU20 6PH (GB)**

(54) **Vectors and methods for assaying the regulation of apolipoprotein ai synthesis.**

(57) The present invention is a method of screening for biological or chemical substances which regulate the synthesis of apolipoprotein AI, the major protein component of high density lipoprotein. Increasing the level of apolipoprotein AI thereby raises the levels of serum high density lipoprotein. A host cell is transformed with a recombinant DNA vector which comprises the regulatory region of the apolipoprotein AI gene operably linked to a reporter gene. The transformed host cell is then exposed to various substances and analyzed for the expression of the reporter gene. The invention further comprises recombinant DNA vectors and host cells.

EP 0 516 443 A1

This invention is in the general area of molecular biology. more particularly it relates to vectors and methods useful in identifying substances which regulate production of apolipoprotein AI, thereby raising serum high density lipoprotein levels.

Coronary artery disease and related complications are the leading causes of death in the United States. The first type of lesion seen, called fatty streaks, are grossly visible, raised, yellow areas which consist of subendothelial foam cells (lipid filled cells derived from macrophages and smooth muscle cells) and some leukocytes. The second type of atherosclerotic lesion, which causes narrowing of the vessel to thrombosis and calcification, is the fibro-fatty plaque. A typical plaque consists of a fibrous cap composed of smooth muscle cells, a few leukocytes and dense extracellular material. A cellular area beneath the cap generally consists of macrophages, foam cells, smooth muscle cells, leukocytes, cellular debris, extracellular lipid, cholesterol crystals and calcium deposits. The cholesterol that accumulates in both of these types of atherosclerotic lesions originates primarily in plasma lipoproteins, predominantly low density lipoprotein (LDL).

Prevention and reversal of cholesterol-mediated vascular damage are significant goals for all health care professionals. One major area of concern centers around the ability of high density lipoprotein (HDL) to facilitate the clearance of cholesterol from the body, thereby preventing and/or reversing the deleterious effects of cholesterol deposition on the vascular wall. HDL mediates reverse cholesterol transport by first taking up excess unesterified cholesterol from the tissue. The enzyme lecithin cholesterol acyltransferase then esterifies the scavenged cholesterol, which then moves into the interior of the HDL molecule. The esterified cholesterol is next transported to other lipoproteins such as very low density lipoprotein, low density lipoprotein and intermediate-density lipoproteins, which, in turn are taken up by specific receptors within the liver. The liver processes the lipoprotein molecules and the cholesterol is secreted into the bile. Apolipoprotein AI is the major protein component of HDL and its transcription rate correlates with its synthesis rate, which is correlated with serum HDL levels. . Because of the pivotal role played by HDL in reverse cholesterol transport, the discovery of substances that upregulate the synthesis of apolipoprotein AI is a key factor in controlling cardiovascular disease.

The present invention is a method of screening for substances which regulate the synthesis of apolipoprotein AI, the major protein component of high density lipoprotein. A host cell is initially transformed with a recombinant DNA vector that comprises the regulatory region of the apolipoprotein AI gene. This regulatory region is linked to a reporter gene in such a manner as to allow expression of the reporter gene upon stimulation of the regulatory region. The transformed host cell is cultured and exposed to various substances and then analyzed for the expression of the reporter gene. The invention also comprises various vectors and transformed host cells useful in the screening method. In the past, the systematic search for substances which regulate apolipoprotein AI synthesis has been hindered due to the time and expense in performing laborious tissue culture and in vivo, screening experiments. This invention allows for the quick and efficient screening of large numbers of substances which may be useful in regulating the synthesis of apolipoprotein AI and high density lipoprotein.

For purposes of the present invention, as disclosed and claimed herein, the following terms are defined below.

Apolipoprotein AI - the major proteinaceous component of high density lipoprotein.

ApoAI - the apolipoprotein AI regulatory region found on the approximately 2.4 kb NdeI to HindIII fragment of plasmids pALC4 and pALC6.

ApR - the ampicillin-resistant phenotype or gene conferring same.

Cloning - the process of incorporating a segment of DNA into a recombinant DNA cloning vector.

Gene - any DNA sequence that encodes a polypeptide, inclusive of that DNA encoding the start and stop codons.

HmR - the hygromycin-resistant phenotype or gene conferring same.

Luc - the firefly luciferase gene

Recombinant DNA Cloning Vector - any autonomously replicating or integrating agent that comprises a DNA molecule to which one or more additional DNA segments can be or have been added.

Recombinant DNA Expression Vector - any recombinant DNA cloning vector comprising a promoter and associated insertion site, into which a DNA sequence that encodes a gene can be inserted and expressed.

Recombinant DNA Vector - any recombinant DNA cloning or expression vector.

Regulatory Region - a segment of DNA which contains a promoter and possibly other elements which may be stimulated or inhibited to allow or prevent transcription of DNA into RNA.

Restriction Fragment - any linear DNA generated by the action of one or more restriction enzymes.

Reporter Gene - any gene, other than the one naturally found associated with the regulatory region, which may be used to detect the stimulation of the regulatory region.

Sensitive Host Cell - a host cell that cannot grow in the presence of a given antibiotic or other toxic compound without a DNA segment that confers resistance thereto.

Transformant - a recipient host cell that has undergone transformation.

Transformation - the introduction of DNA into a recipient host cell.

Figure 1 is a restriction site and function map of plasmid pALC4. For purposes of this disclosure, the Figures are not drawn exactly to scale.

Figure 2 is a restriction site and function map of plasmid pALC6.

The present invention is a method of screening for substances that regulate the synthesis of apolipoprotein AI, said method comprising the steps of:

a) transforming a eukaryotic host cell with a recombinant DNA vector comprising

i) the regulatory region of the apolipoprotein AI gene, and

ii) a reporter gene operably linked to said regulatory region of said apolipoprotein AI gene,

b) culturing said host cell of step a) under conditions suitable for growth of said host cell,

c) exposing said host cell of step b) to various substances to be tested, and

d) analyzing said host cell of step c) for the expression of said reporter gene.

The present invention also concerns vectors and recombinant DNA host cells useful in the method set forth above.

The apolipoprotein regulatory region of the present invention was initially cloned from $P_3$ UCLA DNA using the polymerase chain reaction cloning procedure with primers that create an NdeI restriction site at the 5′ end of the amplified sequence and a HindIII restriction site at the 3′ end of the amplified sequence. The apolipoprotein AI regulatory region resides within a 2.4 kilobase NdeI to HindIII restriction fragment now found in plasmid pALC4 and pALC6. The vectors of the present invention comprise this restriction fragment in conjunction with a wide variety of reporter genes. Examples of such reporter genes are the gene encoding human growth hormone, the gene encoding chloramphenicol acetyltransferase, the gene encoding betagalactosidase, the gene encoding alkaline phosphatase and the gene encoding luciferase. Any of these reporter genes may be cloned into a vector of the present invention by isolating the gene on the proper restriction fragment then cloning said restriction fragment into the vector in such a manner that the reporter gene is controlled by the regulatory region found on the 2.4 kb NdeI to HindIII restriction fragment.

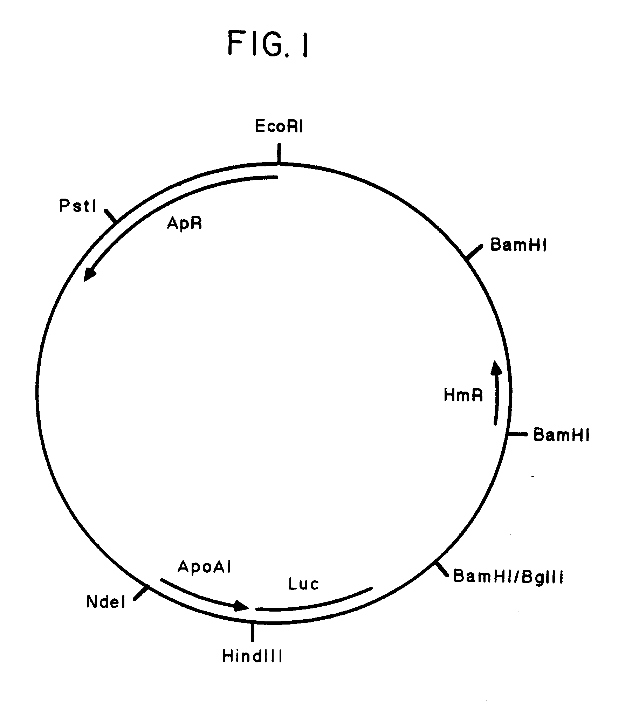

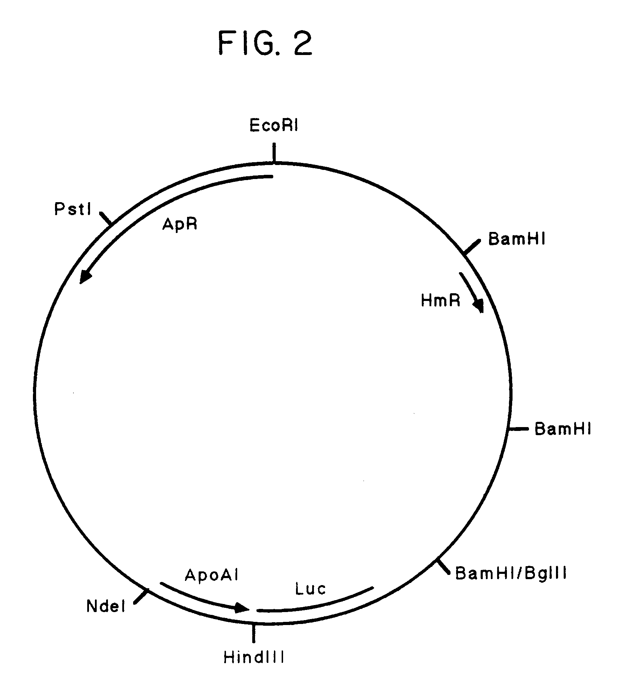

One example of a plasmid of the present invention is plasmid pALC4. Plasmid pALC4 can be conventionally isolated from E. coli K12 DH5α/pALC4 which was deposited and made part of the permanent stock culture collection of the Northern Regional Research Laboratory (NRRL), Peoria, Illinois, on march 13, 1991. A culture of E. coli K12 DH5α/pALC4 can be obtained from the NRRL under the accession number B-18783. A restriction site and function map of plasmid pALC4 is presented in Figure 1 of the accompanying drawings. Another example of a plasmid of the present invention is plasmid pALC6. Plasmid pALC6 was also deposited and made part of the permanent stock culture collection of the NRRL on march 13, 1991. A culture of E. coli K12 DH5α/pALC6 can be obtained from the NRRL under the accession number B-18784. A restriction site and function map of plasmid pALC6 is presented in Figure 2 of the accompanying drawings. Plasmids pALC4 and pALC6 were constructed using a backbone from plasmid pSV2-hyg (NRRL B-18039). Plasmids pALC4 and pALC6 each contain the apolipoprotein AI regulatory region, the firefly luciferase coding region, an SV40 splice site, an SV40 polyadenylation site, an E. coli origin of replication, an ampicillin resistance-conferring gene and a hygromycin resistance-conferring gene.

The firefly luciferase coding sequence of each of these plasmids is positioned in such a manner that the expression of the coding sequences is driven by the regulatory mechanisms found in the 2.4 kb NdeI to HindIII restriction fragment. The skilled artisan will readily recognize that any reporter gene can be cut out on any compatible restriction fragment and placed in a plasmid in such a manner that the expression of said reporter gene is controlled by the regulatory element of the apolipoprotein AI gene. For example, United States Patent No. 4,363,877, the entire teaching of which is herein incorporated by reference, discloses the amino acid and nucleotide sequences for human growth hormone. The DNA sequence encoding human growth hormone can be isolated, purified and ligated to DNA linkers which are compatible with the HindIII restriction site. This fragment can then be ligated into a plasmid containing the apolipoprotein AI regulatory region. The resultant plasmid will then drive the expression of human growth hormone when transformed into a eukaryotic cell line and treated with a substance which up-regulates the apolipoprotein AI regulatory region. Human growth hormone production can be assayed using a wide variety of techniques known to the skilled artisan.

The host cells used in the method of the present invention may also be drawn from a wide variety of sources. Such host cells may include but are not limited to the Caco 2 cell line, T29 cell line, the SK-CO-1 cell line, the Hep3B cell line and the HepG2 cell line. The Caco 2 cell line is available from the American Type Culture Collection in Rockville, maryland, under the accession number ATCC HTB37. The HT29 cell line is available from the ATCC under the accession number HTB38. The SK-CO-1 cell line is available from the ATCC under the accession number HTB39. The Hep3B cell line is available from the ATCC under the accession number HB8064 while the HepG2 cell line is also available from the ATCC under the accession number HTB8065. While these and many other cell lines are useful in the method of the present invention the HepG2 cell line is most preferred.

Once the vector of the present invention has been transformed into a host cell the cells may be cultured, then exposed to a wide variety of substances to be tested. The substances may be chemical or biological in origin and are in no way to be limited by structure or function. After exposure of the host cell to the substance to be tested, the host cell or the supernatant drawn from the culture of the host cell may then be analyzed to see if the expression of the reporter gene has been upregulated by the presence of the test compound or substance. This analysis may be carried out by a wide variety of different tests. For example, if a structural gene such as the human growth hormone gene is used as a reporter gene then one may test for the upregulation of the apolipoprotein regulatory region by exposing the cells to labeled antibodies raised against human growth hormone. The skilled artisan recognizes that a positive reaction to this immunological analysis will indicate that the test substance upregulated the apolipoprotein AI regulatory region. If the betagalactosidase gene is used as a reporter gene, one need only test for the ability of the host cell or the supernatant drawn from the host cell to perform the enzymatic functions normal for betagalactosidase. If the luciferase gene is used as a reporter gene then one merely need test for an increase in bioluminance arising from contact or exposure of any test element that has upregulated the apolipoprotein regulatory region. For simplicity, ease and convenience, the luciferase gene is most preferred to practice the invention.

Plasmids pALC4 and pALC6 both contain the hygromycin resistance conferring gene so that transformed cells may be easily and speedily detected and cultured. If one uses a plasmid of the present invention which does not contain such a selectable marker, the host cell should also be co-transfected or transformed with a second plasmid which contains some selectable marker by which transformed cells may be identified. The hygromycin resistance conferring gene found on plasmids pALC4 and pALC6 may be oriented in either the clockwise or counter clockwise direction. In plasmid pALC6, the hygromycin resistance conferring gene is found oriented in the direction opposite from the orientation of the apolipoprotein AI regulatory element and reporter gene. On the other hand, in plasmid pALC4 the hygromycin resistance conferring gene is found running in the same orientation as the apolipoprotein AI regulatory region and reporter gene.

The skilled artisan will recognize that negative control plasmids are also quite useful when practicing the present invention. For instance, a plasmid which does not contain a regulatory region that can be upregulated is quite useful to use as a negative control. Most specifically, a regulatory region drawn from the human albumin gene is useful as a negative control in that the promoter found in this regulatory region is not upregulated in the same manner as is the apolipoprotein AI promoter found in the regulatory region of the present invention.

The following examples more fully describe the vectors, methods, compounds and recombinant organisms of the present invention. Those skilled in the art will recognize that the particular reagents, equipment and procedures described in the examples are merely illustrative and do not limit the present invention.

### Example 1

#### Isolation of Plasmid pALC4

Lyophils of E. coli K12 DH5α/pALC4 are obtained from the Northern Regional Research Laboratory, Peoria, Illinois 61604, under the accession number NRRL B-18783. The lyophils are decanted into tubes containing 10 ml LB medium (10 g Bacto-tryptone, 5 g Bacto-yeast extract, and 10 g NaCl per liter; pH is adjusted to 7.5) and incubated two hours at 37°C, at which time the cultures are made 50 μg/ml in ampicillin and then incubated at 37°C overnight.

A small portion of the overnight culture is placed on LB-agar (LB medium with 15 g/l Bacto-agar) plates containing 50 μg/ml ampicillin in a manner so as to obtain a single colony isolate of E. coli K12 DH5α-/pALC4. The single colony obtained was inoculated into 10 ml of LB medium containing 50 μg/ml ampicillin and incubated overnight at 37°C with vigorous shaking. The 10 ml overnight culture was inoculated into 500 ml LB medium containing 50 μg/ml ampicillin and incubated at 37°C with vigorous shaking until the culture reached stationary phase.

The following procedure is adapted from Maniatis et al., 1982, Molecular Cloning (Cold Spring Harbor Laboratory).

The cells were harvested by centrifugation at 4000 g for 10 minutes at 4°C, and the supernatant was discarded. The cell pellet was washed in 100 ml of ice-cold STE buffer (0.1 m NaCl; 10 mM Tris-HCl, pH 7.8; and 1 mM EDTA). After washing, the cell pellet was resuspended in 10 ml of Solution 1 (50 mM glucose; 25 mM Tris-HCl, pH 8.0; and 10 mM EDTA) containing 5 mg/ml lysozyme and left at room temperature for 10 minutes. Twenty ml of Solution 2 (0.2 N NaOH and 1% SDS) were then added to the lysozyme-treated cells, and the solution was gently mixed by inversion. The mixture was incubated on ice for 10 minutes.

Fifteen ml of ice-cold 5 m potassium acetate, pH 4.8, were added to the lysed-cell mixture and the solution mixed by inversion. The solution was incubated on ice for 10 minutes. The 5 M potassium acetate solution was prepared by adding 11.5 ml of glacial acetic acid to 28.5 ml of water and 60 ml of 5 M potassium acetate; the resulting solution is 3 M with respect to potassium and 5 M with respect to acetate.

The lysed cell mixture was centrifuged in a Beckman SW27 (or its equivalent) at 20,000 rpm for 20 minutes at 4°C. The cell DNA and debris formed a pellet on the bottom of the tube. About 36 ml of supernatant were recovered, and 0.6 volumes of isopropanol were added, mixed, and the resulting solution left at room temperature for 15 minutes. The plasmid DNA was collected by centrifugation at 12,000 g for 30 minutes at room temperature. The supernatant was discarded, and the DNA pellet was washed with 70% ethanol at room temperature. The ethanol wash was decanted, and the pellet was dried in a vacuum desiccator. The pellet was then resuspended in 8 ml of TE buffer (10 mM Tris-HCl, pH 8.0, and 1 mM EDTA).

Eight grams of CsCl were added to the DNA solution. About 0.8 ml of a 10 mg/ml solution of ethidium bromide in water were added for each 10 ml of CsCl-DNA solution. The final density of the solution was about 1.55 g/ml, and the ethidium bromide concentration was about 600 μg/ml. The solution was transferred to a Beckman Type 50 centrifuge tube, filled to the top with paraffin oil, sealed, and centrifuged at 45,000 rpm for 24 hours at 20°C. After centrifugation, two bands of DNA were visible in ordinary light. After removing the cap from the tube, the lower DNA band was removed by using a syringe with a #21 hypodermic needle inserted through the side of the centrifuge tube.

The ethidium bromide was removed by several extractions with water-saturated 1-butanol. The CaCl was removed by dialysis against TE buffer. After extractions with buffered phenol and then chloroform, the DNA was precipitated, washed with 70% ethanol, and dried. About 1 mg of plasmid pACL4 was obtained and stored at 4°C in TE buffer at a concentration of about 1 μg/μl. A restriction site and function map of plasmid pALC4 is presented in Figure 1 of the accompanying drawings. Plasmid pALC6 is isolated from E. coli K12 DH5α/pALC6 (NRRL B-18784) in substantial accordance with the above teaching. A restriction site and function map of plasmid pALC6 is presented in Figure 2 of the accompanying drawings.

Example 2

Transformation of HepG2 Cells

The transformation procedure described below refers to HepG2 cells as the host cell line; however, the procedure is generally applicable to most eukaryotic cell lines. HepG2 cells are obtained from the ATCC under the accession number ATCC HB8065. The procedure set forth below is a modified procedure using the Lipofectin™ Reagent sold by Bethesda Research Laboratories Life Technologies, Inc. (BRL) of Gaithersburg, maryland 20877 U.S.A. The Lipofectin™ Reagent is a 1:1 (w/w) liposome formulation of N-[1-(2,3-dioleyloxy)propyl]-N,N,N-trimethyl-ammon

ium chloride (DOTMA) and dioleoyl phosphatidylethanolamine (DOPE) in water.

The growth media used to culture the HepG2 host cells is 3:1 SE media, which comprises three parts DMEM media mixed with one part F12 media and 10% Fetal Bovine Serum (FBS). DMEM media, F12 media and FBS can all be purchased from Gibco, Inc. Approximately 5x10$^6$ HepG2 cells were plated onto a 100 millimeter tissue culture dish in 10 milliliters 3:1 SE media. The cells were incubated overnight in a humidified 5-10% carbon dioxide environment at 37°C. The next morning, the dish was washed twice with 5 ml of Reduced Serum MEM which is available also from BRL. The dish was then covered with 5 ml of Reduced Serum MEM in preparation for the transformation.

The DNA (either plasmid pALC4 or pALC6) was linearized by digestion with either restriction enzyme PvuI or Asp700 which are available from New england Biolabs or BRL. The digested DNA was purified by chloroform extraction and ethanol precipitation. Approximately 10μg purified linearized DNA was resuspended in 100μl sterile water. Fifty microliters of the Lipofectin™ Reagent was mixed with fifty microliters of sterile water then added to the linearized DNA mixture. The Lipofectin™ mixture was kept in a polystyrene tube to prevent excess adherence to the tube wall. The Lipofectin™/DNA solution was gently mixed then left at room temperature for 15 minutes.

The 200 μl Lipofectin™/DNA transformation mixture was added to the cells, gently swirled, then left at 37°C for 6 hours. Five milliliters of Reduced Serum MEM containing 20% FBS was added to the plate to bring the final concentration of FBS to 10%. The plate was incubated for 24 hours at 37°C. The old media was removed and fresh 3:1 SE media plus 10% FBS was added. The fresh media also contained 600 μg/ml hygromycin in order to add selective pressure to the cells, thereby selecting only for transformed cells containing the hygromycin resistance conferring gene found on the plasmid DNA.

Media was changed every two to three days until colonies appeared on the plates. Colonies generally appear after 10 to 14 days. After the appearance of colonies, the selective pressure was reduced by lowering the amount of hygromycin in the media to 400 μg/ml. Resistant colonies were then selected and replated on separate dishes and allowed to grow to approximately 85% confluence before being assayed following the procedure set forth in Example 3.

Example 3

Assay for the Regulation of the Apolipoprotein AI Regulatory Region

The stably transformed cells from Example 2 were suspended in media and about one-half million cells were placed per well in a 12-well microtiter plate. The cells were then allowed to grow overnight at 37°C. The next day serial dilutions of the various compounds to be tested in the assay system were prepared and placed in the various microtiter wells in 3:1 SE plus 0.025% bovine Excyte™ (Miles Laboratories). One set of wells was left with no added substance in order to act as a negative control. Another set of wells was treated with serial dilutions of 17-β-estradiol (Sigma). 17-β-estradiol has been found to act as a positive upregulator of the human apolipoprotein AI regulatory elements. The cells were then incubated again overnight at 37°C.

Lysis buffer was prepared which comprises 25 mM glycylglycine, 15 mM magnesium sulfate, 4 mM EGTA, 15 mM potassium phosphate (pH 7.8), 1 mM DTT and 1 mM ATP. The media was aspirated from the cells. The cells were washed one time in Dulbecco's Phosphate Buffered Saline and then 200 microliters of the lysis buffer was added to each of the microtiter wells. After gentle mixing, 75 microliters was withdrawn from each microtiter well and added to 400 microliters of fresh lysis buffer. To each sample was then added 25 microliters of 1 mM luciferin (Boehringer). Each serial dilution was then assayed in an LKB 1251 luminometer to obtain the luminesence value of each reaction. The data may then be plotted as light units per microgram of protein versus the serial dilution value at micrograms per milliliter. Following this procedure it has been demonstrated that β-estradiol is an efficient upregulator of the human apolipoprotein AI regulatory element in a dose/response fashion.

## Claims

1. A method of screening for substances that regulate the synthesis of apolipoprotein AI, said method comprising the steps of:
   a) transforming a eukaryotic host cell with a recombinant DNA vector comprising
      i) the regulatory region of the apolipoprotein AI gene, and
      ii) a reporter gene operably linked to said regulatory region of said apolipoprotein AI gene,
   b) culturing said host cell of step a) under conditions suitable for growth of said host cell,
   c) exposing said host cell of step b) to various substances to be tested, and
   d) analyzing said host cell of step c) for the expression of said reporter gene.

2. The method of Claim 1 wherein the reporter gene is the luciferase gene.

3. The method of Claim 2 wherein the host cell is a HepG2 (ATCC HB8065) cell.

4. The method of Claim 3 wherein the method of analyzing the expression of said reporter gene is luminescence.

5. A recombinant DNA vector that comprises
   (a) the regulatory region of the apolipoprotein AI gene, and
   (b) a reporter gene operably linked to said regulatory region of said apolipoprotein AI gene.

6. The recombinant DNA vector of Claim 5 wherein the reporter gene is the luciferase gene.

7. The recombinant DNA vector of Claim 6 that is selected from the group consisting of plasmid pALC4 and plasmid pALC6.

8. A host cell transformed with a vector of Claim 5.

9. The host cell of Claim 8 that is selected from the group consisting of a HepG2/pALC4 cell and a HepG2/pALC6 cell.

10. The host cell of Claim 8 that is selected from the group consisting of Escherichia coli DH5α/pALC4 (NRRL B-18783) and Escherichia coli DH5α/pALC6 (NRRL B-18784).

# FIG. I

# FIG. 2

**European Patent Office**

# EUROPEAN SEARCH REPORT

Application Number

EP 92 30 4902

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | WO-A-9 101 379 (ONCOGENE SCIENCE)<br>* the whole document *<br>specially<br>* claims 49,57,77,84 *<br>--- | 1-10 | C12Q1/66<br>C12N15/65<br>C12N1/21<br>C12N9/02<br>C12N15/70<br>C07K15/16 |
| X | TRENDS IN BIOTECHNOLOGY.<br>vol. 6, January 1988, CAMBRIDGE GB<br>pages 23 - 27;<br>A. T. SCHAUER: 'Visualizing gene expression with luciferase fusions'<br>* the whole document *<br>--- | 1-10 | |
| A | WO-A-9 010 710 (DELTA BIOTECHNOLOGY LMT)<br>--- | | |
| A | EP-A-0 267 703 (FARMITALIA CARLO ERBA)<br>--- | | |
| E | EP-A-0 496 027 (ROBIT RESEARCH & DEVELOPMENT CO.)<br>* the whole document *<br><br>----- | 1-10 | |

**TECHNICAL FIELDS SEARCHED (Int. Cl.5)**

C12Q
C12N
C07K

The present search report has been drawn up for all claims

| Place of search | Date of completion of the search | Examiner |
|---|---|---|
| THE HAGUE | 01 SEPTEMBER 1992 | MOLINA GALAN E. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document